# EUROPEAN PATENT SPECIFICATION

(11) **EP 2 753 370 B1**
(45) Date of publication and mention of the grant of the patent: **06.01.2016**
(21) Application number: 12755873.2
(22) Date of filing: 06.09.2012
(51) Int. Cl.: A61L 17/00, A61B 17/06, A61L 17/14, A61L 31/10, A61L 31/14, A61L 31/16, A61F 2/00

(54) **FLOCKED MEDICAL DEVICE AND METHODS FOR MANUFACTURING THE DEVICE**
BEFLOCKTE MEDIZINISCHE VORRICHTUNG UND VERFAHREN ZUR HERSTELLUNG DER VORRICHTUNG
DISPOSITIF MÉDICAL FLOQUÉ ET PROCÉDÉ DE FABRICATION DU DISPOSITIF

(30) Priority: 07.09.2011 EP 11180418
(43) Date of publication of application: 16.07.2014
(73) Proprietor: Aesculap AG, 78532 Tuttlingen/Donau (DE); B. Braun Surgical, S.A., 08191 Rubi (Barcelona) (ES)
(72) Inventor: ODERMATT, Erich, CH-8200 Schaffhausen (CH); PFEIFFER, Ruth Verena, E-08173 Sant Cugat del Vallès (Barcelona) (ES)
(74) Representative: Patentanwälte Ruff, Wilhelm, Beier, Dauster & Partner mbB
(86) International application number: PCT/EP2012/067411
(87) International publication number: WO 2013/034641

(56) References cited:
- EP-A1- 2 229 918
- WO-A1-2010/127874
- US-A1- 2002 049 503

## Description

The present invention relates to a flocked medical device, methods for producing a flocked medical device and a surgical combination comprising a flocked medical device.

Wound closure surgery provides for several types of wound closure devices and methods. Typically, sutures, staples, surgical tapes and tissue adhesives are employed. Employment of most of these wound closure devices is time consuming, and requires considerable manual dexterity, carefulness, and patience. Further, conventional wound closure devices may take a long time to provide effect wound closure, or may yield cosmetically unappealing results.

Other typical wound closure devices are wound drapes for tissue adaptions, synthetic textiles or industrial prepared biological tissues used for tissue substitutions or tissue enforcement. These devices must be typically fixed to or within the tissue that is to be addressed. As regards fixation, self-fixating medical devices as further detailed in the following paragraph may be employed.

Such medical devices represent a class of devices which is advantageously not necessarily dependent on knotting or suturing in order to obtain a secure wound closure. Thus, such devices may advantageously facilitate a knot- or sutureless fixation ("self-fixation") after application. Knot-or sutureless devices typically comprise one or more spaced barbs, which project from the devices' surface along their longitudinal direction. Generally, the barbs are produced by cuts into the device. Such devices may be in particular configured as sutures or textile devices such as textile meshes.

Barbed devices are, by way of example, known from WO 2010/127874 A1, WO 2010/127875 A1, WO 2010/052006 A1, EP 2 229 918 A1, and US 2002/0077661 A1.

Due to their specific production, barbs generally cause at least a portion-like reduction of device diameter. However, reducing device diameter is principally associated with impairing the structural integrity, and thus with the risk of weakening the mechanical stability of the devices, thereby complicating a secure wound closure and/or undermining other effects which shall be achieved by the devices.

In view of the foregoing, the object underlying the present invention is therefore to make available a new class of medical device which in particular circumvents withdrawals known from devices of the prior art. The medical device according to the present invention shall in particular allow for an optimized fixation, in particular knotless or even sutureless fixation, within a patient's body, preferably without having impaired mechanical properties.

This object is accomplished by a medical device according to independent claim 1. Preferred embodiments thereof are reflected in dependent claims 2 to 16. Further, the invention relates to methods for manufacturing a medical device according to independent claims 17 and 18. Furthermore, a surgical combination having the features of claim 19 is encompassed by the present invention. The subject matter and wording, respectively of all claims is hereby incorporated into the description by explicit reference.

According to a first aspect of the invention, this object is solved by a medical device comprising at least one device body and a flock material. The term "at least one device body" as used according to the invention means either one device body or a plurality of device bodies, i.e. at least two or more device bodies.

The present invention is based on the surprising finding that flocking technology may be successfully applied to medical devices. Flocked medical devices represent a new versatile class of devices exhibiting numerous advantages in relation to conventional devices:
- Advantageously, flocking a medical device does basically not impair the mechanical characteristics of the device. This is in particular beneficial in respect of secure wound closing.
- Further, by choosing the flock material, in particular the diameter, length and/or titer of the flock material, the surface appearance of the medical device may be influenced. For example, a high length, low diameter and/or low titer of the flock material may yield a device having a cushion-like, smooth or soft surface. This may be desirable in order to prevent or reduce irritation and/or erosion of tissue. In contrast, a low length, large diameter and/or high titer of the flock material may deliver a device having a stiffer surface, thereby aiding fixation, in particular self-fixation, of the device.
- In general, flocking a medical device is typically associated with an enlargement of the device's surface. The enlarged surface advantageously contributes to enhanced friction forces which improve fixation and adhesion of the device within a patient's body. For example, device fixation may be improved by enhanced friction and in particular adhesion forces. This may in turn require no further or at least less fixation steps such as the employment of sutures. Thus, dexterity of the device may be increased, while operation time may be decreased.
   Due to the flock material, the friction forces may be enhanced to such an extent that the application of additional fixating means such as sutures, staples, adhesives, or the like is not mandatory. In other words, it may be within the scope of the present invention that the medical device is a knotless or self-fixating device. Insofar, the input of additional foreign material into the body of a patient which may cause undesired foreign body reactions can be avoided, too.
   Moreover, an enlarged surface may aid a finishing of the device with additives such as active agents, medicaments, and/or cells or liquids including such additives. In that regard, the flock material may function as a retaining structure or reservoir for additives, while the additives may reside in superficial interstices between the flock material. Further, an enlarged surface may act as a soaking reservoir for liquids, in particular for additive such as active agents and/or cells including liquids.
   Specifically, a release of retained additives such as active agents may be controlled by specific flock characteristics such as flock material, flock length, flock diameter and/or flock titer.
- Further advantages which are associated with an enlarged surface relate to better tissue integration and/or protection of the device depending on the structure and type of the flock material.

The term "at least one device body" as used according to the present invention may refer to one device body, i.e. a single device body, or to a plurality of device bodies, i.e. to two or more device bodies. In case that the at least one device body relates to a plurality of device bodies, all device bodies or only a part thereof may exhibit features and advantages as detailed in any of the following embodiments.

The term "flock material" as used according to the present invention defines a material which may be applied on a substrate such as a device body by means of a flocking technique.

Preferably, the medical device additionally comprises an adhesive layer, in particular a cured adhesive layer.

The adhesive layer may be a partially or completely layer, in particular coat, encase, envelope, ensheath, surround or wrap, the at least one device body.

The flock material is preferably attached to the adhesive layer.

Advantageously, the adhesive layer facilitates attachment of the flock material onto the surface, typically onto the exterior surface, of the at least one device body.

Thus, in a further preferred embodiment, the flock material is attached to the at least one device body by means of an adhesive layer.

The flock material may be partly, in particular only partly, incorporated into the adhesive layer.

In particular, it may be preferred according to the invention that the flock material is not incorporated into the at least one device body. In other words, it may be preferable that the flock material does not penetrate or invade the at least one device body.

According to a further embodiment, the flock material is incorporated into the adhesive layer over a length between 0.5 and 95 %, in particular 3 % and 50 %, preferably 5 and 20 %, in relation to its total length, i.e. the total length of the flock material. Depending on the extent of incorporation of the flock material into the adhesive layer, characteristics of the medical device such as dexterity, resistance, stiffness or the like may be adjusted in a targeted fashion.

In a further preferred embodiment, the adhesive layer is formed as a non-textile layer, in particular as a coating, sheath, foil, film, membrane, foam, gel, particularly hydrogel, or paste. The embodiment as a coating or sheath is especially preferred according to the present invention.

According to a further embodiment, the medical device comprises a core-sheath structure. Preferably, the core is provided by the at least one device body, while the sheath is preferably provided by the adhesive and adhesive layer, respectively.

However, it may be also conceivable according to the invention that the adhesive layer is present as a textile structure. Specifically, the adhesive layer may be present in the form of a fibre or filament. Moreover, the adhesive layer may be formed as a plurality of fibres or filaments.

More specifically, the adhesive layer may be formed as a monofilament, pseudo monofilament or multifilament. For example, the adhesive layer may be present as an intertwined, twisted, textured or braided multifilament. According to the invention it may be especially preferred for the adhesive layer to be formed as a multifilament.

According to a further embodiment, the adhesive layer is formed as a two-dimensional or three-dimensional textile structure. The textile structure may be, by way of example, a braided, knitted, woven and/or felted structure.

According to a more specific embodiment, the adhesive layer may be configured as a mesh, in particular as a flat mesh or as a three-dimensional mesh.

In a further embodiment, the adhesive layer may be configured as velour slings.

Further, the adhesive layer may have a proportion between 0.1 and 90 % by weight, in particular between 0.5 and 50 % by weight, preferably between 1 and 25 % by weight, related to the total weight of the medical device.

Furthermore, the adhesive layer may have a thickness between 1 and 500 µm, in particular between 3 and 300 µm, preferably between 5 and 100 µm.

In principle, the adhesive layer may be absorbable, partially absorbable or non-absorbable. In other words, the adhesive layer may include or be made of an absorbable material, a partially absorbable material or a non-absorbable material. Examples of suitable materials are provided in the following.

Typically, the material is a polymer. A polymer may be a synthetic polymer or biopolymer, i.e. a naturally occurring polymer.

The term "material" or "polymer" as used according to the invention may relate to one, i.e. single material or polymer, or to a mixture of different materials or polymers.

Specifically, the material may be a copolymer.

The term "copolymer" as used according to the present invention, relates to a polymer which is composed of at least two different monomer units. Thus, the term "copolymer" may encompass, by way of example, bipolymers (polymer composed of two different monomer units), terpolymers (polymers composed of three different monomer units) or tetrapolymers (polymers composed of four different monomer units).

Further, a copolymer according to the present invention may be selected from the group consisting of a random copolymer, an alternating copolymer, a block or segmented copolymer, particularly a triblock terpolymer, a graft copolymer, and combinations thereof.

The adhesive layer may be distinct or proprietary from the at least one device body. The distinctness may be based on different chemical and/ or physical properties such as different materials, cross-linking, solubility or swelling characteristics towards liquids, in particular aqueous liquids such as aqueous dispersions, aqueous solutions and/or aqueous suspensions, thermal stability, melting point, crystallinity, or the like.

More specifically, the adhesive layer may include or be made from a different material than the at least one device body. Alternatively, the adhesive layer may include or be made of the same material as the at least one device body.

In a further advantageous embodiment, the adhesive layer may include or be made of a material, typically a polymer, preferably a thermoplastic polymer, having a lower melting temperature than a material, typically a polymer, which is included in the at least one device body or of which the at least one device body is made. Thus, a specific activation of the adhesive layer may be achieved by applying temperatures which allow for melting the material of the adhesive layer but not the material of the at least one device body. This advantageously does not impair the basic integrity of the at least one device body, while allowing the flock material to be attached onto the at least one device body. Moreover, the melting temperature of the material which is included in the adhesive layer or of which the adhesive layer is made may be lower than the melting temperature of the flock material.

It may be further within the scope of the present invention to use the same material, in particular polymer, for the adhesive layer and the at least one device body wherein the material, in particular polymer, for the adhesive layer is a lower melting material, in particular polymer, in comparison to the material, in particular polymer, for the at least one device body.

In a further embodiment, the adhesive layer may include or be made of a material which exhibits adhesive or sticky properties upon having contact with a liquid, in particular physiological or medicinal liquid. Useful liquids may be water, aqueous solutions, buffer solutions, electrolyte solutions, and combinations thereof. In this embodiment, the material is preferably a biopolymer such as a protein and/or polysaccharide. With respect to useful biopolymers, reference is made to the following description.

In principle, the adhesive layer may include or be made of any material. In that regard, the adhesive material is not limiting the present invention provided that the material allows for attaching the flock material onto the at least one device body, particularly may be activated to exhibit adhesive or sticky properties towards a flock material.

According to a useful embodiment, the adhesive layer includes or is made of a glue or a hot melt.

According to a more specific embodiment, the adhesive layer may include or be made of a polymer, particularly a non-absorbable or partly absorbable polymer which is preferably selected from the group consisting of hot melts, in particular based on polyamide, polyester, polyolefin, polyethylene, amorphous poly-α-olefin, polybutene-1, ethylene-vinyl acetate, polyurethanes such as thermoplastic polyurethanes or the like, polyvinyl alcohol, polyvinyl acetate, polyvinyl chloride, polyvinyl propionate, polyacrylates, cyanoacrylates, poly-α-olefin, polyurethanes, modified vinylacetat copolymer, polyester-elastomer, polyurethane-elastomer, vinylpyrrolidon-vinylacetat copolymer, polyethylene glycol based hydrogels, resins such as synthetic resins, waxes like bee waxes and/or synthetic waxes, and combinations thereof.

Useful resins may be made of urea, epoxide, polyurethane, melamin, phenol, polyester, polyamide, vinylester, and combinations thereof.

Alternatively or in combination, the adhesive layer may include or be made of an absorbable polymer which is selected from the group consisting of polyhydroxyalkanoates, polyglycolide, polylactide, polydioxanone, polyhydroxybutyrate, poly-3-hydroxybutyrate, poly-4-hydroxybutyrate, polytrimethylenecarbonate, poly-ε-caprolactone, polysaccharides, cellulose derivates such as alkylcelluloses, methylcellulose, hydroxyalkylcelluloses, hydroxymethylcellulose, hydroxyethylcellulose, hydroxylpropylcellulose, carboxyalkylcellulose, carboxymethylcellulose, copolymers thereof, salts thereof, stereoisomers therefore, and combinations thereof.

In a further embodiment, the adhesive layer includes or is made of a biopolymer, in particular a protein and/or polysaccharide, preferably selected from the group consisting of structural proteins, extracellular proteins, fibrous proteins, globular proteins, oxidized or non-oxidized polysaccharides, amino group bearing polysaccharides, aldehyde group bearing polysaccharides, mucopolysaccharides, salts thereof, stereoisomers thereof, and combinations thereof.

Preferably, the adhesive layer includes or is made of a biopolymer selected from the group consisting of collagen, gelatine, elastin, reticulin, fibronectin, laminin, fibrin, albumin, starch, amylose, amylopectin, dextran, dextrin, cellulose, chitin, chitosan, hyaluronic acid, dextran sulfate, heparin, heparan sulfate, chondroitin sulfate, dermatan sulfate, salts thereof, stereoisomers thereof, and combinations thereof.

In a further embodiment, the adhesive layer may include or be made of any mixture of materials as mentioned in the previous embodiments.

Further, it may be also within the scope of the present invention that the at least one device body is free of a distinct or proprietary adhesive layer, in particular of an adhesive layer as described in any of the preceding embodiments.

Instead, the flock material may be directly attached to the at least one device body.

More specifically, it may be preferably that the flock material is directly incorporated into the at least one device body, in particular into a superficial layer thereof. In other words, the flock material may penetrate or invade the at least one device body, particularly a superficial layer thereof.

According to a further preferred embodiment, the at least one device body, in particular a superficial layer thereof, is at least partly, in particular completely, made of a material, in particular polymer, which acts as an adhesive, preferably upon activation. Having regard to a useful material, in particular polymer, reference is made to the materials, in particular polymers, which have been mentioned for the adhesive layer.

In a further embodiment, the flock material is attached to an adhesive layer formed onto the at least one device body, in particular as outlined in any of the preceding embodiments, or as an alternative, to a superficial layer of the at least one device body via non-covalent forces such as adhesion forces, Van-der-Waals forces, or the like.

The term "activation" as used according to the present invention includes any process such as melting, heating, soaking, liquefying, humidifying, irradiating such as ultraviolet or infrared irradiating, exposing to ultrasonic waves, exposing to chemicals or the like, transferring an adhesive layer or a superficial layer of the at least one device body into an adhesive or sticky state allowing for attaching the flock material onto the at least one device body.

With respect to the flock material, any material may be used according to the present invention as long as the material may be applied onto the at least one device body by means of a flocking technique.

The flock material may in principle only partially or completely coat or cover the at least one device body. In particular, the at least one device body may be circumferentially coated or covered by the flock material. A complete coating or covering may be preferred inasmuch as undesired adhesions, for example with bowel, can be avoided. Further it may be preferred that pores of the at least one device body are unoccupied, i.e. not coated or sealed with the flock material. This advantageously helps to prevent the formation of seromas. A complete flocking of the at least one device body can be achieved by turning the at least one device body constantly around during the flocking process.

In an especially preferred embodiment, the flock material is formed as fibres, in particular as cut, pulverized, crushed and/or milled fibres.

Further, the flock material may be present as a particulate material such as a powder, pulver or granulate.

The selection of the size of the flock material may follow different sieving fractions.

The flock material may have a length between 0.1 and 15 mm, in particular between 0.2 and 10 mm, preferably between 0.3 and 5.0 mm.

Furthermore, the flock material may have a diameter between 5 and 800 µm, in particular 7 and 300 µm, preferably between 10 and 150 µm.

In a further embodiment, the flock material may protrude from the at least one device body, in particular from an adhesive layer which is formed onto the at least one device body, over a length between 10 µm and 14 mm, in particular between 0.1 and 9.0 mm, preferably between 0.2 and 4.8 mm.

In a further embodiment, the flock material may have a linear mass density between 1 and 1000 dtex, in particular between 1.5 and 500 dtex, preferably between 2 and 200 dtex. The dimension "dtex" (deci tex) means a linear mass density of 1 g per 10000 m length of the flock material.

Typically, the flock material protrudes from the surface of an adhesive layer or from a superficial layer of the at least one device body. Preferably, the flock material may protrude perpendicularly or essentially perpendicularly from the adhesive layer or superficial layer of the at least one device body. The term "essentially perpendicular" as used herein may include aberrations up to 5 degrees from the right angle. However, it may be also conceivable that the flock material protrudes in an acute or oblique angle from the adhesive layer or superficial layer of the at least one device body. An acute or oblique angle according to the present invention preferably relates to an angle which is more than 0 degrees but less than 85 degrees.

In a further useful embodiment, the flock material may comprise a two-dimensional, in particular pattern-like, disposition onto the at least one device body, in particular onto an adhesive layer formed onto the at least one device body. The disposition is preferably selected from the group consisting of a linear disposition, a staggered disposition, a helical or spiral disposition, a meander-like disposition, a serpentine-like disposition, a sinus-like disposition, an annular disposition, and combinations thereof.

Alternatively, a random or irregular disposition of the flock material onto the at least one device body, in particular onto an adhesive layer formed onto the at least one device body, may be also within the scope of the present invention.

For example, a helical or spiral disposition may be realized by means of a filament, typically a monofilament, which is helically or spirally wound onto the at least one device body and which may act as an adhesive layer for the flock material. Alternatively, a helical pattern may be realized using a braided device body whose filaments are partially made of a thermoplastic polymer having a lower melting point than the polymers of other filaments of the braided device body. Thus, the thermoplastic filaments may be activated into adhesive filaments which advantageously may yield a helix-shaped flock pattern.

Further, the flock material may be present onto the at least one device body in a unidirectional or multidirectional, in particular bidirectional, disposition. A unidirectional disposition as used herein means a disposition where the flock material is oriented in one direction, while a bidirectional disposition relates to a disposition where the flock material is oriented in two, preferably two opposing directions.

The dispositions as described in the preceding embodiments may advantageously act as fixation means and/or marker means.

According to a further example, a two-dimensionally shaped at least one device body may be covered, at least one side thereof, with a mask with open areas, wherein the open areas are arranged in a certain pattern. Flocking the masked at least one device body and subsequent removal of the mask, yields a medical device comprising a pattern of flock material which corresponds to the arrangement of open areas of the mask.

According to a further embodiment, the flock material comprises a section-like or repeating disposition, preferably an annular or ring-like disposition, onto the at least one device body, in particular onto an adhesive layer being formed onto the at least one device body. Annular dispositions of the flock material may be present at one or both ends and/or in the middle of the at least one device body. Advantageously, annular dispositions may act as control, marker, reference or stopper structures for the surgeon.

In principle, the flock material may be absorbable, partially absorbable or non-absorbable. In other words, the flock material may include or be made of an absorbable, a partially absorbable or non-absorbable material.

According to the invention, it may be especially preferred that the flock material includes or is made of an absorbable material. Due to absorption of the flock material, tissue remodelling may be elicited, thereby assisting a secure attachment of the device within a patient's body.

According to an advantageous embodiment, the flock material may include or be made of a swellable material, in particular a material that is swellable in water or aqueous media such aqueous solutions, aqueous suspensions, aqueous buffers and/or body liquids such as blood. Having regard to useful materials, reference is made to the materials, in particular polymers, mentioned in the following.

Specifically, the flock material may include or be made of a polymer, particularly a synthetic polymer and/or a biopolymer. Specifically, the flock material may include or be made of a copolymer. Besides, the flock material may include or be made of a terpolymer, in particular a triblock terpolymer.

More specifically, the flock material may include or be made of a synthetic polymer which is preferably selected from the group consisting of polyolefines, polyamides, polyesters, polyurethanes, in particular thermoplastic polyurethanes, polyhydroxyalkanoates, copolymers thereof, and combinations thereof.

Alternatively or in combination, the flock material may include or be made of a biopolymer or a related polymer thereof, preferably a protein and/or polysaccharide.

According to a more specific embodiment, the flock material is selected from the group consisting of structural proteins, extracellular proteins, fibrous proteins, globular proteins, enzymes, antibodies, blood clotting factors, oxidized or non-oxidized polysaccharides, amino group bearing polysaccharides, aldehyde group bearing polysaccharides, mucopolysaccharides, salts thereof, stereoisomers thereof, and combinations thereof.

According to a more specific embodiment, the flock material may include or be made of a material which is selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, rayon, silk, in particular spider silk, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylenecarbonate, poly-ε-caprolactone, collagen, gelatine, elastin, reticulin, fibronectin, laminin, fibrin, fibrinogen, albumin, starch, amylose, amyloptectin, dextran, dextrin, cellulose, cellulose derivates such as alkyl cellulose, methylcellulose, hydroxyalkylcellulose, hydroxymethylcellulose, hydroxyethylcellulose, hydroxypropylcellulose, carboxyalkylcellulose, carboxymethylcellulose, chitin, chitosan, hyaluronic acid, dextran sulfate, heparin, heparan sulfate, chondroitin sulfate, dermatan sulfate, copolymers thereof, salts thereof, stereoisomers thereof, and combinations thereof.

A preferred copolymer for the flock material is made of glycolide and lactide, in particular in a weight ratio from 9:1 to 1:9, in particular 7:3 to 3:7.

A preferred terpolymer for the flock material may be a terpolymer, in particular a triblock terpolymer, made of glycolide, trimethylenecarbonate and ε-caprolactone. Such a terpolymer is, by way of example, commercially available under the trademark Monosyn^{®}.

In a further preferred embodiment, the flock material may be a physiologically active material. The term "physiologically active material" as used herein preferably means a material that is able to exercise a beneficial effect in vivo under a medical point of view, preferably in respect of an optimized, in particular accelerated, wound healing.

In a more specific embodiment, the flock material may include or be made of an active agent such as a cosmetic agent, biological agent, pharmaceutical agent, medicinal agent or combinations thereof.

Useful active agents are preferably selected from the group consisting of antimicrobial agents, in particular antibiotics, disinfectants, oncological agents, anti-scarring agents, ant-inflammation agents, pain-killing agents, blood clotting agents, growth factors, cellular differentiating factors, cellular adhesion factors, cellular recruiting factors, cell receptors, cell-binding factors, cytokines, peptides, structural or extracellular proteins such as collagen, polysaccharides such as hyaluronic acid, oligonucleotides, polynucleotides, DNA, RNA, salts thereof, stereoisomers thereof, and combinations thereof.

According to a more specific embodiment, the active agent is selected from the group consisting of fibroblast growth factor (FGF), transforming growth factor (TGF), platelet derived growth factor (PDGF), epidermal growth factor (EGF), granulocyte-macrophage colony stimulation factor (GMCSF), vascular endothelial growth factor (VEGF), insulin-like growth factor (IGF), hepatocyte growth factor (HGF), interleukin-1-B (IL-1B), interleukin-8 (IL-8), nerve growth factor (NGF), bone morphogenetic proteins (BMPs) such as BMP-1 and/or BMP-2, and combinations thereof.

Suitable antimicrobial agents may be selected from the group consisting of biguanide, polyhexamethylene biguanide (PHMB), trichlosan, gentamicine, copper, zinc, silver, gold, salts thereof, stereoisomers thereof, and combinations thereof.

A preferred anti-scarring agent is an angiotensin-converting enzyme inhibitor such as captopril.

It may be further within the scope of the present invention that the flock material includes or is made of a mixture of materials as mentioned in any of the preceding embodiments in respect of the flock material.

The flock material may have a proportion between 0.01 and 50% by weight, in particular between 0.5 and 20 % per weight, preferably between 1.0 and 10 % by weight, related to the total weight of the medical device.

In principle, the flock material may represent one type of flock material or different types of flock material. Different types of flock material may be different in respect of material, length, height, diameter, density, dispositions or the like. In that regard, reference is explicitly made to present description.

The at least one device body may be further present as a flat or two-dimensionally formed body. For example, the at least one device body may be formed as a sheet, foil, sheath, membrane, pad, patch, or the like.

Alternatively, the at least one device body may be present as a three-dimensionally shaped or three-dimensionally formed body such as three-dimensionally formed mesh or such as a vascular graft.

According to a further embodiment, the at least one device body may be formed as a hollow body, i.e. a formed body having a cavity which is contained by a wall defining an interior and an exterior surface. For example, the at least one device body may be designed as a tube or hose. In this embodiment, the flock material is normally formed onto the exterior surface of the hollow body, in particular of the tube or hose. Thus, the flock material advantageously may mimic a velour structure of a vascular graft such as a polyester vascular graft, thereby promoting tissue integration. Further, the cavity may be advantageously filled with active agents and/or cells, or alternatively, with liquids, and particular solutions, or pastes including active agents and/or cells. Thus, the cavity may be used as drug and/or cell deposit. With respect to useful active agents, reference is made to the agents that have already been enumerated in respect of the flock material. With respect to useful cells, reference is made to the following description.

Preferably, the at least one device body may comprise a textile structure.

Specifically, the at least one device body may comprise a thread or a plurality of threads. The thread and threads, respectively may be configured as fibre(s), monofilament(s), pseudo monofilament(s) or multifilament(s).

More specifically, the at least one device body may comprise a thread having a core-sheath structure or a plurality of such threads, wherein preferably each thread has a core sheath structure. The sheath is preferably provided by an adhesive layer or a material that may act as an adhesive upon activation. With respect to useful materials for the core and sheath, in particular the adhesive layer or material which may act as an adhesive upon activation, reference is made in its entirety to the present description.

For example, the at least one device body may be formed as a textile body. Preferably, the at least one device body is formed as a textile fabric, in particular as a planar, two-dimensional, pre-shaped, three-dimensional or hollow textile fabric. For a better tissue in-growth, the textile fabric may have a velour structure.

In a more specific embodiment, the at least one device body is formed as a textile fabric, in particular as a mesh, having velour slings which advantageously act as an adhesive layer according to the present invention and thus may exhibit sticky properties upon activation.

Due to a further useful embodiment, the medical device comprises a device body and a flock material, wherein the device body is shaped as a long-term absorbable mesh, in particular made of poly-4-hydroxybutyrate and the flock material is formed as fibres made of a terpolymer, in particular triblock terpolymer, made of glycolide, trimethylene carbonate and ε-caprolactone. In this embodiment, a superficial layer of the device body, i.e. a superficial poly-4-hydroxybutyrate layer of the mesh, serves as an adhesive layer.

According to a preferred embodiment, the at least one device body is a textile fabric which is selected from the group consisting of a woven fabric, a mesh or open meshed fabric, a knitted fabric, in particular a warpknitted fabric, a non-woven fabric such as a sprayed non-woven fabric, a needled non-woven fabric, a water-jet non-woven fabric, (electro-)spun non-woven fabric or the like, a fibrous web, a felt, composites thereof, and combinations thereof.

Moreover, the at least one device body may have a porous, in particular open-porous, structure. Preferably, as already mentioned, pores of the at least one device body are unoccupied, i.e. not coated or sealed with the flock material.

According to an especially preferred embodiment, the at least one device body is a mesh or an open meshed fabric, in particular a textile mesh or textile open meshed fabric, preferably a knitted mesh or knitted open meshed fabric.

The term "composite" as used according to the present invention refers to a formed body which is composed of at least two different structures, such as two or more different textile structures, two or more different non-textile structures or a combination of textile structures and non-textile structures. With respect to useful non-textile structures, it is referred to the following description.

A preferred composite that may serve as at least one device body to be flocked according to the present invention relates to an implant, comprising a first layer and a second layer, wherein said first layer comprises a membrane-like structure and said second layer comprises a sponge-like structure. The sponge-like structure preferably comprises directional and/or interconnected pores. The implant is especially useful for repairing a cartilage defect. In that regard, said first layer is preferably facing the synovial space and said second layer is preferably located towards bone. The implant, in particular its sponge-like structure, may be advantageously seeded with cells, in particular chondrocytes. Such an implant is commercially available under the name NOVOCART^{®} 3D. For further details and advantages of the implant, reference is made to the document WO 2006/045330 A1 whose disclosure is hereby made to the content of the present description by explicit reference.

In a further embodiment, the at least one device body may be formed as a non-textile body, in particular selected from the group consisting of a membrane, foam, sponge, tape, patch, sheet, pad, patch, layer, coating, film, foil, composites thereof, and combinations thereof.

In principle, the at least one device body may be absorbable, partially absorbable or non-absorbable. In other words, the at least one device body may include or be made of an absorbable, a partially absorbable or a non-absorbable material.

Specifically, the at least one device body may include or be made of a polymer, particularly a synthetic polymer and/or a biopolymer. More specifically, the at least one device body may include or be made of a copolymer. Besides, the at least one device body may include or be made of a terpolymer, in particular a triblock terpolymer.

Specifically, the at least one device body may include or be made of a synthetic polymer which is selected from the group consisting of polyolefines, polyamides, polyesters, polyurethanes, in particular thermoplastic polyurethanes, polyhydroxyalkanoates, copolymers thereof, and combinations thereof.

Alternatively or in combination, the at least one device body may include or be made of a biopolymer such as a protein and/or polysaccharide or a related polymer thereof. Preferably, the at least one device body includes or is made of a biopolymer which is selected from the group consisting of structural proteins, extracellular proteins, fibrous proteins, globular proteins, enzymes, antibodies, oxidized or non-oxidized polysaccharides, amino group bearing polysaccharides, aldehyde group bearing polysaccharides, mucopolysaccharides, salts thereof, stereoisomers thereof, and combinations thereof.

More specifically, the at least one device body may include or be made of a material which is selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, rayon, silk, in particular spider silk, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxbutyrate, polytrimethylenecarbonate, poly-ε-caprolactone, collagen, gelatine, elastin, reticulin, fibronectin, laminin, fibrin, albumin, starch, amylose, amylopectin, dextran, cellulose, cellulose derivatives such as methylcellulose, hydroxymethylcellulose, hydroxyethylcellulose, hydroxylpropylcellulose, carboxymethylcellulose, chitin, chitosan, hyaluronic acid, dextran sulfate, heparin, heparan sulfate, chondroitin sulfate, dermatan sulfate, copolymers thereof, salts thereof, stereoisomers thereof, and combinations thereof.

As regards polyvinyl alcohol as possible material for the at least one device body, non-absorbable and/or absorbable polyvinyl alcohol may be used. Further, cross-linked, particularly chemically or physically cross-linked, polyvinyl alcohol may be used. Furthermore, polyvinyl alcohol having a high molecular weight may be used.

A preferred copolymer for the at least one device body is a copolymer made of glycolide and lactide, in particular in a weight ratio from 9:1 to 1:9, in particular 7:3 to 3:7.

A preferred terpolymer for the at least one device body may be a terpolymer, in particular a triblock terpolymer, made of glycolide, trimethylenecarbonate and ε-caprolactone. As already mentioned, such a terpolymer is, by way of example, commercially available under the trademark Monosyn^{®}.

The medical device in whole may be in principle absorbable, partially absorbable or non-absorbable.

Further, the at least one device body, the flock material and a preferably provided adhesive layer (or a material being capable of acting as an adhesive upon activation) according to the present invention may include or be made of the same material or different materials. With respect to useful materials, in particular polymers, reference is made in its entirety to the previous description.

It may be further within the scope of the present invention that the at least one device body includes or is made of a mixture of materials as mentioned in any of the preceding embodiments in respect of the at least one device body.

In the following, a number of especially preferred embodiments for the medical device according to the present invention are described:
According to a preferred embodiment, the medical device comprises a device body and a flock material, wherein the device body is a partially absorbable mesh and the flock material is preferable absorbable. The mesh may be composed of threads having a core-sheath structure, wherein the core is made of a non-absorbable material and the sheath is made of an absorbable material or vice versa. Preferably, the core is made of polypropylene, while the sheath is made of poly-4-hydroxybutyrate. The flock material is preferably present as a powdered terpolymer, particularly triblock terpolymer, preferably made of glycolide, trimethylene carbonate and ε-caprolactone.

According to a further useful embodiment, the medical device comprises a device body and a flock material, wherein the device body is a non-absorbable mesh and the flock material is also non-absorbable. Preferably, the mesh is composed of threads having a core-sheath structure, wherein the core is preferably made of polypropylene, while the sheath is made of non-absorbable polyvinyl alcohol. The flock material is preferably formed as polypropylene fibres.

In a further advantageous embodiment, the medical device comprises a device body and a flock material, wherein the device body is a non-absorbable mesh, for example made of polypropylene, and wherein the flock material is formed as fibres made of a copolymer made of glycolide and lactide or made of a terpolymer, in particular triblock terpolymer, made of glycolide, trimethylene carbonate and ε-caprolactone. Preferably, the flock material is present only on one side of the mesh, while the opposing side is unflocked. After implantation, the unflocked side of the mesh is directed towards the abdominal cavity, while the flocked side is directed to the abdominal wall. Due to the hydrolysable nature of the flock material, existing non-anatomical adhesions which may occur during initial residence time will disappear with time. On the flocked side of the mesh, the flock material is conveniently arranged in a unidirectional disposition.

In a further preferred embodiment, the medical device comprises a device body, an adhesive layer and a flock material, wherein the device body is a non-absorbable mesh, the adhesive layer is formed as a film or tape made of an absorbable material and wherein the flock material is also made of an absorbable material. In that embodiment, it is especially preferred that the mesh is made of polypropylene, the film or tape is made of poly-4-hydroxybutyrate and the flock material is present as threads made of polyvinyl alcohol.

According to a further advantageous embodiment, the medical device comprises a device body, an adhesive layer and a flock material, wherein the device body is present as a non-absorbable mesh, for example made of polypropylene, the adhesive layer is made of poly-4-hydroxybutyrate and the flock material is formed as fibres made of a terpolymer, in particular triblock terpolymer, preferably made of glycolide, trimethylene carbonate and ε-caprolactone.

According to a further useful embodiment, the medical device comprises a device body, an adhesive layer and a flock material, wherein the device body is a non-absorbable mesh such as a polypropylene mesh, the adhesive layer is a glue based on polyurethane and the flock material is present as a powdered protein such as collagen.

A further preferred embodiment provides a medical device comprising a device body, an adhesive layer and a flock material, wherein the device body is formed as a non-absorbable mesh such as a mesh made of polyester like polyethylene terephthalate, the adhesive layer is formed as an absorbable film such as a film made of poly-4-hydroxybutyrate and the flock material is powdered polyvinyl alcohol.

According to a further preferred embodiment, the medical device comprises a device body, an adhesive layer and a flock material, wherein the device body is a non-absorbable mesh such as a polypropylene mesh, the adhesive layer is present as an absorbable film, for example a film made of poly-4-hydroxybutyrate, and the flock material is an absorbable material being preferably formed as powdered gelatine.

A further useful embodiment provides a medical device comprising a device body, an adhesive layer and a flock material, wherein the device body is formed as a non-absorbable mesh, for example as a mesh made of polypropylene or polyvinylidene difluoride, the adhesive layer is formed as a non-absorbable and in particular perforated film, for example made of non-absorbable polyvinyl alcohol, and wherein the flock material is an absorbable material, preferably being present as powdered absorbable polyvinyl alcohol.

According to a further useful embodiment, the medical device comprises a device body and a flock material, wherein the device body is a membrane or felt including or being made of a protein and wherein the flock material is formed as fibres including or being made also of a protein. The protein for the membrane and felt, respectively, may be the same protein as the protein for the flock material. Alternatively, the protein for the membrane and felt, respectively, may be different from the protein for the flock material. More specifically, the device body may be a membrane or felt including or being made of collagen or gelatine, while the flock material is configured as fibres including or being made also of collagen or gelatine. According to an alternative specific embodiment, the membrane or felt includes or is made of collagen or gelatine, while the flock material is formed as fibres including or being made of a different protein such as thrombin and/or fibrinogen. Such a membrane and felt, respectively is especially useful for being applied as a wound healing patch.

According to a further embodiment, the medical device, in particular the at least one device body, the flock material and/or a preferably provided adhesive layer according to the present invention, may be finished or equipped with additives such as active agents, drugs, pharmaceutical agents, cells, dies, softening agents, radiopaque agents, marking agents, nanoparticles, or the like. With respect to useful agents reference is explicitly made to the previous description, in particular to the active agents mentioned in respect of the flock material.

Useful cells may be selected from the group consisting of fibroblasts, chondrocytes, osteocytes, osteoblasts, adipocytes, miocytes, neurones, astrocytes, oligodentrocytes, hepatocytes, pancreatic cells, progenitor cells thereof, stem cells, in particular mesenchymal stem cells, adult stem cells, umbilical stem cells, fetal stem cells, thereof, and combinations thereof.

Further, the cells may be autologous, allogenic and/or xenogenic cells. However, in order to minimize the risk of immunological responses, cells of autologous origin are especially preferred.

In case that a cell-finished medical device is provided according to the present invention, the cells, in particular as detailed in any of the proceeding embodiments, can be loaded directly before implantation onto and/or into the device, for example by soaking the flock surface of the device with a cell including liquid such as a cell broth. Specifically, the device maybe seeded with cells in the laboratory with the advantage of a better adhesion, adaptation and multiplication of the cell line.

Further, the at least one device body and/or flock material may have a circular and/or non-circular cross-section. Conceivable non-circular cross-sections may be selected from the group consisting of oval cross-section, ellipsoid cross-section, polygonal cross-section such as triangular cross-section, square cross-section, rectangular cross-section, diamond cross-section, pentagonal cross-section, hexagonal cross-section, star-like cross-section, and combinations thereof.

In principle, the medical device may be provided for topological applications. For example, the medical device may be a wound dressing or haemostatic device.

More preferable, the medical device according to the present invention is provided for internal applications.

Specifically, the medical device may be employed in the field of abdominal surgery, laparoscopic surgery, gynaecological surgery, neurosurgery, spinal surgery, orthopaedic surgery, lung surgery and/or plastic surgery.

It is especially preferred that the medical device is an implant, in particular a surgical implant. The implant comprises at least one implant body and a flock material. Having regard to the at least one implant body and the flock material, reference is made in its entirety to the previous description.

Specifically, the medical device may be a wound closure and/or wound healing device, in particular a wound closure and/or wound healing implant.

More specifically, the medical device may be selected from the group consisting of a surgical mesh such as hernia mesh, prolapse mesh, incontinence mesh, shunt, catheter, stent, stent-graft, vascular graft, in particular arterial graft, aortic graft, prosthesis, in particular hip prosthesis, knee prosthesis, fascia substitute, cartilage substitute, cell carrier, anastomotic device, dura mater substitute, dura mater onlay, wound leakage device, wound drainage device, wound dressing and haemostatic patch, in particular based on non-wovens, felts, membranes, foils, or the like.

According to a specific embodiment, the medical device is designed as a wound drainage device, wherein the at least one device body preferably comprises an absorbent body, preferably a sponge or foam, and a drainage tube being attached to the absorbent body. Preferably, the flock material is attached to the absorbent body, typically to an exterior surface thereof. More preferably, the absorbent body may be formed as a two-dimensional, in particular planar, entity such as a membrane, tape, foil, and the like, wherein the flock material is attached to the entity, typically to an exterior surface thereof, on at least one two-dimensional side, particularly on both two-dimensional sides, of the entity.

Further, the medical device, in particular if designed as a textile device such as a textile fabric, may be employed as a filling device, for example for filling aneurysms, in particular brain aneurysms. In contrast to conventional approaches to treat aneurysms (such as clips, coiled wires, adhesives, and the like), the medical device according to the present invention is beneficially inasmuch as it contributes to a significantly reduced risk of aneurysm damage. Insertion of the medical device may be facilitated by appropriate catheters or pushers.

As mentioned at the beginning, the flock material may facilitate a convenient finishing of the medical device with additives such as active agents and/or cells. Thus, it is preferred according to the present invention that the medical device may be present as a drug and/or cell release system.

Further, as also mentioned at the beginning, the flock material may advantageously contribute to a self-fixation of the medical device. Thus, the medical device according to the present invention, is preferably formed as a self-anchoring, self-fixating or knotless medical device, in particular surgical implant.

A second aspect of the present invention relates to a method of manufacturing or producing a medical device, in particular in accordance with the present invention. The method comprises the steps of
a) layering at least one device body with an adhesive,
b) flocking the adhesive-layered at least one device body with a flock material while the adhesive is still active, in particular wet, or upon activation of the adhesive.

The at least one device body may be partially or completely layered with the adhesive.

In a preferred embodiment, step a) is achieved by means of extruding, injecting, casting, screen printing, coating with a doctor blade, immersing, dipping, spraying, brushing, painting, and/or rolling techniques.

Preference of the afore-enumerated application techniques depends on the condition of the adhesive. For example, if the adhesive is made of a meltable material, the adhesive may be applied onto the at least one device body by means of extrusion, in particular by means of sheath extrusion.

In case that the at least one device body is also made of a meltable material, step a) may be achieved by co-extrusion of the at least one device body and the adhesive.

If the adhesive is present as an aqueous liquid, in particular aqueous solution, it may be preferable to layer the at least one device body with the adhesive by means of spraying or, as an alternative, by immersing or dipping the at least one device body into an adhesive including liquid.

In case that the adhesive is present in a paste-like or gel-like, in particular hydrogel-like, condition, it may be more convenient to apply the adhesive onto the at least one device body by means of brushing, painting, screen printing, coating with a doctor blade, rolling, or the like.

The term "while the adhesive is still active" relates according to the invention to any condition of the adhesive under that the adhesive exhibits adhesive or sticky properties allowing for an attachment of the flock material onto the at least one device body during a flocking process.

Preferably, the adhesive is activated by means of heating, liquefying such as melting, humidifying, wetting, soaking, swelling, partial dissolving, partial dispersing, irradiating such as ultraviolet or infrared irradiating, exposing to ultrasound waves, exposing to chemicals, or the like.

Flocking is normally understood as a process of depositing flock material being preferably configured as milled, crushed or pulverized fibers, i.e. small fiber particles (called flock) onto a surface of a substrate.

The flocking may be in general achieved by means of spreading, spraying, blowing, vibration and/or electrostatic techniques.

According to the present invention, it is especially preferred to flock the adhesive-layered at least one device body by means of electrostatic flocking technology.

Electrostatic flocking relates to a flocking technique which is normally aided by a high-voltage electric field. Typically, the surface of a substrate which is to be flocked is coated with an adhesive. Then, the flock material is applied electrostatically onto the substrate while the adhesive is still wet. Electrostatic flocking may be performed in a specific flocking apparatus. Typically, the flocking apparatus is given a negative charge, while the substrate is earthed. By doing so, the flock material normally flies vertically onto the substrate attaching to the previously applied adhesive.

If electrostatic flocking is employed to manufacture the medical device according to the present invention, the flock material, preferably formed as fibers, may be straightened in the electrostatic field and in particular may be fixed in an orientated manner onto the at least one device body. Due to a high acceleration of the flock material in the electrostatic field, a good incorporation, in particular penetration or invasion, of the flock material into the adhesive layer may be achieved.

According to the present invention, the at least one device body is preferably applied as an electrode, in particular as an earthed electrode, along with a counter electrode, in particular in the form of a high-voltage electrode. More preferably, the at least one device body is applied as a cathode and the counter electrode is preferably applied as an anode.

The electrodes may have a mutual distance from 2 to 200 cm, in particular from 5 to 100 cm, preferably from 7 to 50 cm.

In a further embodiment, a voltage from 5 to 120 kV (kilo Volt), in particular from 10 to 100 kV (kilo Volt), preferably from 20 to 80 kV (kilo Volt), may be applied.

Advantageously, after flocking the adhesive-layered at least one device body and prior to a drying step, the flock material may be tilted back or over, in particular pressed onto the at least one device body, and/or brushed so as to accomplish specific and desired effects. In particular, the flock material may be evenly melted. Preferably the flock material may be partially melted, thereby yielding flock material, in particular flock fibres, having heads at the free, in particular protruding, ends of the flock material. In other words, according to the present invention it may be advantageously that the flock material, preferably in the form of fibres, comprises heads at the free ends, i.e. at the ends which are not attached to the at least one device body.

With respect to further features and advantages of the manufacturing process, in particular in respect of the medical device, the at least one device body, adhesive or adhesive layer and/or flock material, reference is made in its entirety to the previous description.

A third aspect of the present invention relates to a method of manufacturing or producing a medical device, in particular in accordance with the present invention, comprising the steps of
a) providing at least one device body which is at least partly made of a material which acts as an adhesive upon activation,
b) flocking the at least one device body with a flock material upon activation of the at least one device body's material.

In order to avoid unnecessary repetitions, reference is also made to the previous description with respect to further features and advantages of the manufacturing process, in particular in respect of possible embodiments for steps a) and/or b), process characteristics, process parameters, medical device, at least one device body, material which may act as an adhesive upon activation and/or flock material.

A fourth aspect of the present invention relates to a surgical combination, particularly to a surgical kit, comprising a medical device, in particular according to the present invention, and at least one, in particular one, delivery instrument.

The at least one delivery instrument advantageously serves to deliver, i.e. deploy or place, the medical device to a target area within a patient's body.

The at least one delivery instrument may be, by way of example, formed as a catheter, tube, or the like.

Alternatively, the at least one delivery instrument may be used through a trocar instrument. The instrument may typically comprise a grip portion, a rectilinear portion, typically extending from the grip portion and a head portion. Depending on the instrument type, the head portion may be configured as a linear or arcuate portion.

Advantageously, the at least one delivery instrument may comprise at least one fixation means that allows for attaching or gripping the medical device. Useful attaching means may be, by way of example, configured as an eye, hole or fixation arms. For example, the at least one delivery instrument may have two fixation arms. Thus, a flat-shaped medical device may be fixed between the fixation arms and the device may be wound around the fixation arms. In that condition, the device may be passed through a trocar. After release from the trocar, the device may be unwound from the fixation arms. Advantageously, the device may be fixed by means of its flocked structure to the area of need.

In case that the at least one delivery instrument is formed as a tube or catheter, such attaching means may be configured in the grip portion, in particular in an area where a rectilinear portion emerges from the grip portion, and/or in a head portion of the tube and catheter, respectively.

Besides, the medical device may be attached to the at least one delivery instrument by means of clamping, in particular mechanical clamping, gluing, welding, in particular ultrasound welding, stitching or suturing, stapling, or the like.

With respect to further features and advantages, in particular in respect of the medical device, reference is made in its entirety to the previous description.

Further features and advantages of the invention will become clear from the following description of preferred embodiments in form of a figure, figure description and examples in conjunction with the subject matter of the dependent claims. The individual features can be realized either singularly or severally in combination in one embodiment of the invention. The preferred embodiments merely serve for illustration and better understanding of the invention and are not to be understood as in any way limiting the invention.

The figures schematically show the following:
- Figure 1:: an embodiment of a medical device according to the present invention and
- Figure 2:: a further embodiment of a medical device according to the present invention.

### Figure description

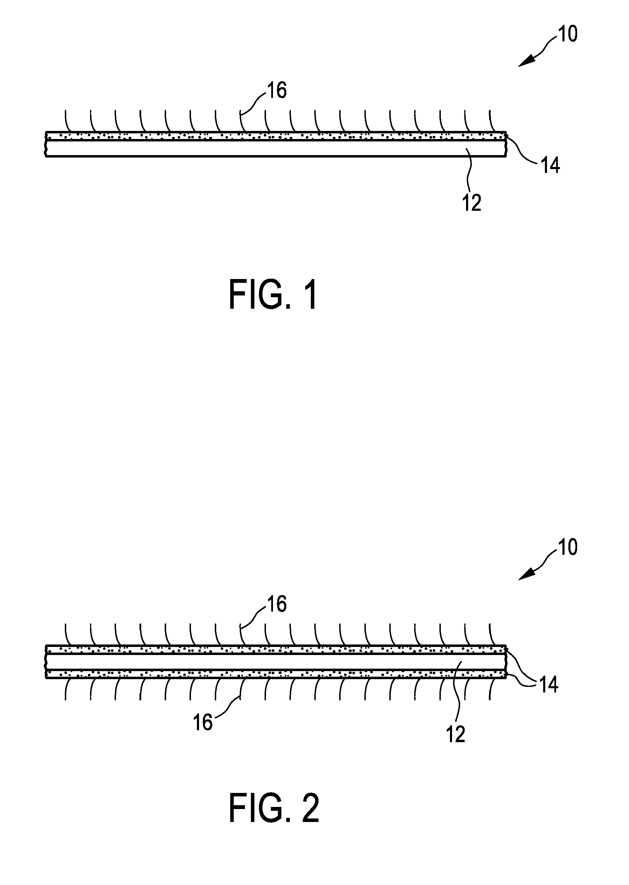

Figure 1 schematically displays a lateral view of a medical device 10 according to the present invention. The medical device 10 comprises a device body 12 which is present in a planar formation, an adhesive layer 14 and a flock material 16. The adhesive layer 14 and the flock material 16 may be present only on one planar side of the device body 12 (as displayed in Figure 1). If the device body 12 is configured as a textile fabric such as a mesh, the medical device 10 is especially useful for hernia management. Advantageously, the device 10 is then implanted such that the flocked side thereof is orientated towards the abdominal cavity, thereby acting as an anti-adhesive barrier, while the unflocked side is directed towards the abdominal wall, allowing for tissue ingrowth and secure fixation of the device 10.

Figure 2 schematically displays a lateral view of a further medical device 10 according to the present invention. The medical device 10 comprises a device body 12 which is present in a planar formation, an adhesive layer 14 and a flock material 16. The adhesive layer 14 and the flock material 16 are present on both planar sides of the device body 12. The flock material 16 being present on the opposing planar sides may be made of the same or different materials. The medical device 10 as depicted in figure 2 may also be advantageously employed for hernia management inasmuch as one planar side is adapted to be located towards the abdominal cavity, while the opposing planar side is adapted to be located towards the abdominal wall. Thus, the flock material 16 that is directed to the abdominal cavity may advantageously act as an anti-adhesive layer, while the flock material 16 that is directed towards the abdominal wall may beneficially contribute to self-fixation of the medical device 10.

With respect to further features and advantages of the medical devices 10, in particular their device body 12, adhesive layer 14 and/or flock material 16, as shown in the figures 1 and 2, reference is made in its entirety to the previous description.

### Examples

### Example 1: Manufacture of a partially absorbable mesh comprising absorbable flock material

It was provided a mesh composed of threads having a core-sheath structure. The core was made of polypropylene. The sheath was made of poly-4-hydroxybutyrate.

For the flocking process, the mesh was transferred into a flocking cabin, where the mesh was flocked with a powdered triblock terpolymer made of glycolide, trimethylene carbonate and ε-caprolactone. The cabin was heated to a temperature of 60 ± 5 °C, thereby allowing the sheath to be molten.

For the flocking process, the mesh was applied as an earthed electrode, along with a high-voltage electrode. The mesh and the high-voltage electrode had a mutual distance of 40 cm. By means of a high-voltage generator (electrostat) a voltage of 60 kV was applied.

Due to the high voltage, an electric field was generated between the mesh to be flocked and the high-voltage electrode.

The flock powder was provided by means of a dosing apparatus which was situated in the flocking cabin.

Upon passing the high-voltage electrode, the powder adapted charges by means of electrostatic induction and was accelerated along the streamlines of the electric field in the direction of the mesh, where the powder stuck to the molten sheath of the mesh threads. Surplus flock powder was continuously sucked off during the flocking process.

Additionally, the mesh was brushed to get rid of non-fixed powder.

A subsequent drying step yielded a polypropylene mesh being flocked with a powder made of a triblock terpolymer as detailed before.

### Example 2: Manufacture of a non-absorbable mesh comprising non-absorbable flock material

A mesh having threads with a core-sheath structure was provided. The core was made of polyethylene terephthalate (PET), while the sheath was made of polypropylene.

For the flocking process, the mesh was transferred into a flocking cabin which was heated to a temperature of about 160 to 165 °C, thereby allowing melting of the polypropylene sheath, while passing through the cabin (online-process).

Immediately upon melting the sheath, the mesh was flocked with polypropylene fibres having a length of about 0.2 mm.

For the flocking process, the mesh was applied as an earthed electrode, along with a high-voltage electrode. The mesh and the high-voltage electrode had a mutual distance of 20 cm. By means of a high-voltage generator (electrostat) a voltage of 60 kV was applied.

Due to the high-voltage, an electric field was generated between the mesh and the high-voltage electrode.

The polypropylene fibres were provided by means of a dosing apparatus which was situated in the flocking cabin.

Upon passing the high-voltage electrode, the fibres adapted charges by means of electrostatic induction and were accelerated along the streamlines of the electric field in the direction of the mesh, where the fibres stuck to the molten sheath. Surplus polypropylene fibres were continuously withdrawn by suction during the flocking process.

The flocked mesh was additionally released from surplus flocks by vacuuming the mesh surface rigorously.

A subsequent drying step yielded a non-absorbable mesh being flocked with fibres made of polypropylene. The polypropylene fibres contributed to a 15- to 20-fold enlargement of the surface of the mesh.

### Example 3: Manufacture of a partially absorbable mesh comprising absorbable flock material

A mesh made of polypropylene was layered with a film tape made of poly-4-hydroxybutyrate.

For the flocking process the layered mesh was transferred into a flocking cabin. For activation of the tape, the flocking cabin was heated to a temperature of 60 ± 5 °C.

Immediately upon activating the tape, the layered mesh was flocked with milled yarns made of uncrosslinked polyvinyl alcohol, wherein the yarns had a length of about 0.5 mm.

The layered mesh was applied as an earthed electrode, along with a high-voltage electrode. The mesh and the high-voltage electrode had a mutual distance of 30 cm. By means of a high-voltage generator (electrostat) a voltage of 50 kV was applied.

Due to the high-voltage, an electric field was generated between the layered mesh to be flocked and the high-voltage electrode.

The milled yarns were provided by means of a dosing apparatus which was also situated in the flocking cabin.

Upon passing the high-voltage electrode, the milled yarns adapted charges by means of electrostatic induction and were accelerated along the streamlines of the electric field in the direction of the layered mesh, where the milled yarns bond to the activated tape. Surplus yarns were continuously withdrawn by suction during the flocking process.

A subsequent drying step yielded a polypropylene mesh being flocked with uncrosslinked polyvinyl alcohol.

According to the above-described process, a polypropylene mesh may be obtained which is characterized in that only one two-dimensional side thereof is flocked with milled yarns made of polyvinyl alcohol and wherein the milled yarns are arranged unidirectionally on that side.

If such a mesh is implanted as an abdominal wall enforcement in such a way that the flocked two-dimensional side of the mesh is directed to the intestinal cavity, post-surgical adhesions can be avoided inasmuch as the milled yarns made of polyvinyl alcohol get into contact with body water, thereby forming an anti-adhesive layer, while the unflocked side of the mesh still guarantees integration via porosity (see figure 1).

### Example 4: Manufacture of a partially absorbable mesh comprising absorbable flock material

A polypropylene mesh was dipped into molten poly-4-hydroxybutyrate, thereby yielding a polypropylene mesh coated with poly-4-hydroxybutyrate. The coating advantageously acted as an adhesive layer upon activation.

For the flocking process, the coated mesh was transferred into a flocking cabin. The cabin was heated to a temperature of 60 ± 5 °C, thereby transferring the coating into a molten condition.

Immediately upon melting the coating, the coated mesh was flocked with fibres made of Monosyn^{®} (length 0.4 mm). The coated mesh was applied as an earthed electrode, along with a high-voltage electrode. The mesh and the high-voltage electrode had a mutual distance of 40 cm. By means of a high-voltage generator (electrostat) a voltage of 60 kV was applied.

Due to the high-voltage, an electric field was generated between the mesh and the high-voltage electrode.

The Monosyn^{®} fibres were provided by means of a dosing apparatus which was situated in the flocking cabin.

Upon passing the high-voltage electrode, the fibres adapted charges by means of electrostatic induction and were accelerated along the streamlines of the electric field in the direction of the mesh, where the fibres stuck to the molten coating. Surplus Monosyn^{®} fibres were continuously sucked off during the flocking process.

A subsequent drying step yielded a coated polypropylene mesh being flocked with Monsyn^{®} fibres.

Such a mesh advantageously exhibits the same effects as regards prevention of post-surgical adhesions and achievement of post-surgical integration as the mesh manufactured according to example 3.

### Example 5: Manufacture of a non-absorbable mesh comprising absorbable flock material

A polypropylene mesh having a small pore section and a large pore section was passed through a wide spray nozzle which was responsible for the formation of a polyurethane coating (Baymedix CH110) onto the mesh. For better wetting purposes, the mesh was plasma activated.

For the flocking process, the coated mesh was immediately transferred into a flocking cabin where the mesh was flocked with powdered collagen immediately upon activation of the polyurethane coating. The collagen was fractioned after different sieving steps.

The mesh to be flocked was applied as an earthed electrode. In addition, a high-voltage electrode was applied. The coated mesh and the high-voltage electrode were mutually spaced from each other in a distance of 20 cm. By means of a high-voltage generator (electrostat) a voltage of 50 kV was applied.

Due to the applied high-voltage, an electric field was generated between the mesh to be flocked and the high-voltage electrode.

The collagen powder was provided by means of a dosing apparatus which was also located in the flocking cabin.

Upon passing the high-voltage electrode, the powder adapted charges by means of electrostatic induction and was accelerated along the streamlines of the electric field in the direction of the coated mesh, where the powder got stick to the activated polyurethane coating. Surplus collagen powder was continuously withdrawn by suction during the flocking process.

A subsequent drying and curing step at ≥ 40 °C for 1 hour yielded a polyurethane coated polypropylene mesh being flocked with powdered collagen.

The powdered collagen covered the small pore section of the mesh more densely than the large pore section. Additionally, the powdered collagen was more perpendicular directed to the electrode for the small pore section.

### Example 6: Manufacture of a partially absorbable mesh comprising absorbable flock material

A mesh made of polypropylene was coated with poly-4-hydroxybutyrate. The coating acted as an adhesive layer upon activation.

For the flocking process, the coated mesh was transferred into a flocking cabin. For activation of the coating, the flocking cabin was heated to a temperature of 60 +/- 5 °C. The temperature in the cabin was sufficient to transfer the coating in a molten and thus sticky condition.

Immediately upon melting the coating, the coated mesh was flocked with powdered non cross-linked polyvinyl alcohol having a molecular weight of 130 kilo Dalton (Mowiol 18-88, Aldrich).

The coated mesh to be flocked was applied as an earthed electrode, along with a high-voltage electrode. The mutual distance between the coated mesh and the high-voltage electrode was 30 cm. By means of a high-voltage generator (electrostat), a voltage of 70 kV was applied.

Due to the high-voltage, an electric field was generated between the coated mesh and the high-voltage electrode.

The powdered polyvinyl alcohol was provided by means of a dosing apparatus which was also situated in the flocking cabin.

Upon passing the high-voltage electrode, the fibres adapted charges by means of electrostatic induction and were accelerated along the streamlines of the electric field in the direction of the coated mesh, where the powder bound to the molten poly-4-hydroxybutyrate coating. Surplus polyvinyl alcohol powder was continuously sucked off during the flocking process.

A subsequent drying step yielded a poly-4-hydroxybutyrate coated polypropylene mesh being flocked with polyvinyl alcohol powder.

### Example 7: Manufacture of a partially absorbable mesh comprising absorbable flock material

A mesh made of polypropylene was coated with a perforated polycaprolacton film having a thickness of 80 µm.

For the flocking process, the coated mesh was transferred into a flocking cabin. In order to activate the perforated film, the flocking cabin had a temperature of 65 °C. This temperature was sufficient to transfer the perforated film into a sticky condition. Due to the melting, the film softened and adapted to the mesh structure.

Immediately upon transferring the film into a sticky condition, the coated mesh was flocked with albumin powder (fraction V, C. Roth GmbH).

The coated mesh was applied as an earthed electrode. Additionally, a high-voltage electrode was applied. The coated mesh and the high-voltage electrode had a mutual distance of 20 cm. By means of a high-voltage generator (electrostat) a voltage of 70 kV was applied.

Due to the applied high-voltage, an electric field was generated between the coated mesh to be flocked and the high-voltage electrode.

The albumin powder was provided by a dosing apparatus which was situated in the flocking cabin.

Upon passing the high-voltage electrode, the powder adapted charges by means of electrostatic induction and was accelerated along the streamlines of the electric field in the direction of the coated mesh, where the powder got stuck to the sticky perforated film. Surplus powder was continuously sucked off during the flocking process.

A subsequent drying step yielded a coated polypropylene mesh being flocked with albumin powder.

### Example 8: Manufacture of an absorbable membrane comprising absorbable flock material

A collagen suspension (22 g/l) was poured into a glass sheet up to 1 cm. With the water evaporating slowly over a couple of days, a tight and stable collagen membrane was formed. Depending on the requested thickness, the process was repeated several times until the requested thickness and stability was achieved. The membrane was covered with a non-conductive template leaving parts of the membrane open.

Collagen membranes resulting from the above-described process had a thickness ranging between 80 µm and 500 µm. For the further process, a membrane having a thickness of 250 µm and a size of 5 x 8 cm was used.

The membrane was sprayed with a 60 °C heated gelatine solution, thereby obtaining a collagen membrane being coated with gelatine.

While the coating was still liquid and warm (ca. 40 °C), the coated mesh was immediately transferred into a flocking cabin (ca. 40 °C) where flocking with powdered thrombin was performed.

For the flocking process, the coated membrane was applied as earthed electrode along with a high-voltage electrode. The coated membrane to be flocked and the high-voltage electrode had a mutual distance of 15 cm. By means of a high-voltage generator (electrostat) a voltage of 60 kV was applied.

Due to the applied high-voltage, an electric field was generated between the coated membrane and the high-voltage electrode.

The powdered thrombin (Sigma Aldrich) was provided by means of a dosing apparatus which was also situated in the flocking cabin.

Upon passing the high-voltage electrode, the powdered thrombin adapted charges due to electrostatic induction and was accelerated along the streamlines of the electric field in the direction of the coated membrane, where the powder bound to the jellied gelatine coating. Thrombin was fixed to the surface of the coated membrane, while gelation of the gelatine coating still occurred. After complete gelation, surplus thrombin was blown from the device.

A subsequent drying step yielded a collagen membrane being flocked with thrombin powder.

The flocked membrane exhibited excellent hemostatic behaviour towards capillary bleedings.

### Example 9: Manufacture of a flocked felt

An oxidized cellulose felt (Surgicel, 5 x 8 cm²) was provided. Under a temperature ranging between 42 and 48 °C, a gelatine solution was casted onto the pre-warmed (ca. 50 C°) felt, thereby forming a thin film onto the felt.

While the gelatine film was still liquid, the film-layered felt was transferred into a flocking cabin where the felt was flocked with fibrinogen powder.

For the flocking process, the film-layered felt was applied as an earthed electrode along with a high-voltage electrode. The film-layered felt to be flocked and the high-voltage electrode had a mutual distance of 15 cm. By means of a high-voltage generator (electrostat), a voltage of 60 kV was applied.

Due to the applied high-voltage, an electric field was produced between the film-layered felt and the high-voltage electrode.

The powdered fibrinogen was provided by means of a dosing apparatus which was situated in the flocking cabin.

Upon passing the high-voltage electrode, the fibrinogen powder adapted charges due to electrostatic induction and was accelerated along the streamlines of the electric field in the direction of the film-layered felt where the powder got stick to the jellied gelatine film which fixed the fibrinogen during the gelation process.

A subsequent drying step yielded a film-layered cellulose felt being flocked with fibrinogen powder.

The device may be beneficially used as a sealing patch, especially for sealing perforated lung tissue or liver tissue.

### Example 10: Manufacture of a flocked pericardium membrane

A native membrane of pericardium was washed and prepared similar to the manufacturing process of the pericardium membrane which is commercially available under the trademark Lyoplant^{®} (B. Braun Melsungen AG).

The wet pericardium membrane (5 x 8 cm²) was coated with the two-component adhesive GLUETISS^{®} (gelatine-resorcinol solution/aqueous glutardialdehyde/glyoxal solution).

Immediately after mixing both components of GLUETISS^{®} onto the membrane, the membrane was transferred into a flocking cabin where the coated membrane was flocked with collagen fibres. The collagen fibres were continuously selected from different sieving fractions and had a length of 1.0 mm.

For the flocking process, the coated membrane was applied as an earthed electrode. Additionally, a high-voltage electrode was applied. The mutual distance between the covered membrane to be flocked and the high-voltage electrode was 20 cm. By means of a high-voltage generator (electrostat) a voltage of 60 kV was applied.

Due to the applied high-voltage, an electric field was generated between the coated membrane and the high-voltage electrode.

The collagen fibres were provided by means of a dosing apparatus which was located in the flocking cabin.

Upon passing the high-voltage electrode, the collagen fibres adapted charges due to electrostatic induction and were accelerated along the streamlines of the electric field in the direction of the coated membrane where the fibres bound to the hardening gelatine glue.

A subsequent drying step yielded a pericardium membrane which was coated with gelatine and flocked with collagen fibres.

The collagen fibres showed a homogeneous distribution over the membrane where the selected collagen fibres protruded perpendicularly from the surface of the membrane and surprisingly resisted to tension and pressure in the wet as well as dry state.

Cell experiments confirmed that cells such as chondrocytes were able to integrate and settle between the collagen fibres, thereby being usable as a cell-seeded implant, for example for repairing cartilage after joint trauma.

### Example 11: Manufacture of a flocked vascular graft

A vascular graft made of polyethylene terephthalate was coated with a heated gelatine solution (50 to 60 °C).

While the coating was still liquid, the coated graft was transferred into a heated flocking cabin where flocking with powdered polyhexamethylene biguanide was performed. The ends of the graft were closed by means of a non-conductive fixation means. During the flocking process, the graft was continuously rotated in order to obtain a densely flocked graft. For the flocking process, the coated vascular graft was applied as an earthed electrode along with a high-voltage electrode. The mutual distance between the vascular graft to be flocked and the high-voltage electrode was 60 cm. By means of a high-voltage generator (electrostat) a voltage of 90 kV was applied.

Due to the applied high-voltage, an electric field was generated between the vascular graft and the high-voltage electrode.

The powdered PHMB was provided by means of a dosing apparatus which was located in the flocking cabin.

Upon passing the high-voltage electrode, the powdered PHMB adapted charges due to electrostatic induction and was accelerated along the streamlines of the electric field in the direction of the coated graft where the powdered PHMB stuck to the wet gelatine coating.

After a subsequent drying step, an antimicrobially finished vascular graft having PHMB powder on its exterior surface was obtained.

## Claims

1. Medical device comprising at least one device body and a flock material, **characterized in that** the medical device additionally comprises an adhesive layer, wherein the flock material is attached to the adhesive layer.

2. Medical device according to claim 1, **characterized in that** the adhesive layer is made of a material, particularly a polymer, having a lower melting temperature than a material, particularly a polymer, of which the at least one device body is made.

3. Medical device according to claim 1 or 2, **characterized in that** the adhesive layer includes or is made of a material selected from the group consisting of hot melts, polyvinyl alcohol, polyvinyl acetate, polyvinylchloride, polyvinyl propionate, polyacrylates, epoxy resins, acrylic resins, resin sizes such as urea resin size, polyurethanes, waxes, polyhydroxyalkanoates, proteins such as collagen, gelatine, elastin, fibronectin and the like, polysaccharides, copolymers thereof, stereoisomers thereof, salts thereof, and combinations thereof.

4. Medical device according to any of the preceding claims, **characterized in that** the flock material is formed as powder or fibres, in particular as crushed or milled fibres.

5. Medical device according to any of the preceding claims, **characterized in that** the flock material is arranged in a pattern-like disposition onto the at least one device body, in particular onto the adhesive layer, wherein the disposition is preferably selected from the group consisting of linear disposition, staggered disposition, helical or spiral disposition, meander-like disposition, serpentine-like disposition, random or irregular disposition, annular disposition, and combinations thereof.

6. Medical device according to any of the preceding claims, **characterized in that** the flock material includes or is made of a material which is selected from the group consisting of polyolefines, polyvinyl alcohols, polyamides, polyimides, pdyesters, polyurethanes, in particular thermoplastic polyurethanes, polyhydroxydkanoates, proteins, polysaccharides, copolymers thereof, stereoisomers thereof, salts thereof, and combinations thereof, wherein the flock material more preferably includes or is made of a material which is selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, rayon, silk, in particular spider silk, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylenecarbonate, poly-ε-caprolactone, proteins such as collagen, gelatine, elastin, reticulin, fibronectin, laminin, fibrin, fibrinogen, albumin, protein such as starch, amylose,amylopectin, dextran, cellulose, methylcellulose, carboxymethylcellulose, chitosan, hyaluronic acid, dextran sulfate, heparin, heparan sulfate, chondroitin sulfate, dermatan sulfate, copolymers thereof, stereoisomers thereof, salts thereof, and combinations thereof.

7. Medical device according to any of the preceding claims, **characterized in that** the flock material includes or is made of an active agent, in particular a cosmetic, pharmaceutical and/or medicinal agent, preferably selected from the group consisting of antibiotics, disinfectants, oncological agents, anti-scarring agents such as captopril, inflammation inhibitors, pain-killing agents, growth factors, cellular differentiating factors, cellular adhesion factors, cellular recruiting factors, cell receptors, cell binding sequences, cytokines, peptides, proteins such as a collagen, lipids, polysaccharides such as hyaluronic acids, oligonucleotides, polynucleotides, DNA, RNA, stereosiomers thereof, salts thereof, and combinations thereof.

8. Medical device according to any of the preceding claims, **characterized in that** the at least one device body includes or is made of a material selected from the group consisting of polyolefines, polyamides, polyesters, polyurethanes, polyhydroxyalkanoates, proteins, polysaccharides, copolymers thereof, stereoisomers thereof, salts thereof, and combinations thereof, wherein the at least one device body more preferably includes or is made of a material which is selected from the group consisting of polyethylene, low-density polyethylene, high-density polyethylene, high-mole-cular-weight polyethylene, ultra-high-molecular-weight polyethylene, polypolypropylene, polyethylene terephthalate, polypropylene terephthalate, polybutylene terephthalate, polyamide 6, polyamide 6-6, polyamide 6-12, polyamide 12, rayon, silk, in particular spider silk, polytetrafluorethylene, polyvinylidene dichloride, polyvinyllidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylenecarbonate, poly-ε-caprolactone, collagen, gelatine, elastin, fibronectin, laminin, fibrin, albumin, starch, amylose, amylopectin, dextran, cellulose, methyl-cellulose, carboxymethylcellulose, chitosan, hyaluronic acid, dextran sulfate, heparin, heparan sulfate, chondroitin sulfate, dermatan sulfate, copolymers thereof, stereoisomers thereof, salts thereof, and combinations thereof.

9. Medical device according to any of the preceding claims, **characterized in that** the at least one device body is formed as a textile fabric which is preferably selected from the group consisting of a woven fabric, a knitted fabric, in particular a warp knitted fabric, a non-woven fabric, in particular a sprayed non-woven fabric, a felt, composites thereof, and combinations thereof.

10. Medical device according to any of the preceding claims, **characterized in that** the at least one device body is a mesh or an open meshed fabric, in particular a textile open meshed fabric, preferably a knitted open meshed fabric.

11. Medical device according to any of the claims 1 to 8, **characterized in that** the at least one device body is formed as a non-textile body, preferably selected from the group consisting of membrane, sheet, patch, pad, foam, sponge, foil, film, coating and combinations, in particular composites, thereof.

12. Medical device according to any of the preceding claims, **characterized in that** the device is a surgical implant.

13. Medical device according to any of the preceding claims, **characterized in that** the medical device is selected from the group consisting of hernia implant, in particular hernia mesh, prolapse implant, in particular prolapse mesh, incontinence implant, in particular incontinence mesh, shunt, catheter, tissue scaffold, stent, stent-graft, vascular graft, in particular arterial graft, aortic graft, hip prosthesis, knee prosthesis, cartilage substitute, fascia substitute, cell carrier, dura mater onlay, anastomotic device, wound dressing and hemostatic patch.

14. Method of manufacturing a medical device according to any of the preceding claims, comprising the steps of
a) layering at least one device body with an adhesive,
b) flocking the adhesive-layered at least one device body with a flock material while the adhesive is still active, in particular wet, or upon activation of the adhesive.

15. Method of manufacturing a medical device according to any of the claims 1 to 13, comprising the steps of
a) providing at least one device body which is at least partly made of a material which acts as an adhesive upon activation,
b) flocking the at least one device body with a flock material upon activation of the at least one device body's material.

16. Surgical combination, in particular surgical kit, comprising a medical device according to any of the claims 1 to 13, and at least one delivery instrument.

## Patentansprüche

1. Medizinprodukt, umfassend mindestens einen Produktkörper und ein Flockmaterial, **dadurch gekennzeichnet, dass** das Medizinprodukt zusätzlich eine Klebstoffschicht umfasst, wobei das Flockmaterial an der Klebstoffschicht angebracht ist.

2. Medizinprodukt nach Anspruch 1, **dadurch gekennzeichnet, dass** die Klebstoffschicht aus einem Material, insbesondere einem Polymer, gebildet ist, das eine niedrigere Schmelztemperatur aufweist als ein Material, insbesondere ein Polymer, aus dem der mindestens eine Produktkörper gebildet ist.

3. Medizinprodukt nach Anspruch 1 oder 2, **dadurch gekennzeichnet, dass** die Klebstoffschicht ein Material beinhaltet oder daraus gebildet ist, das ausgewählt ist aus der Gruppe bestehend aus Schmelzklebstoffen, Polyvinylalkohol, Polyvinylacetat, Polyvinylchlorid, Polyvinylpropionat, Polyacrylaten, Epoxidharzen, Acrylharzen, Harzleimen wie Harnstoffharzleim, Polyurethanen, Wachsen, Polyhydroxyalkanoaten, Proteinen wie Kollagen, Gelatine, Elastin, Fibronectin und dergleichen, Polysacchariden, Copolymeren davon, Stereoisomeren davon, Salzen davon und Kombinationen davon.

4. Medizinprodukt nach einem der vorhergehenden Ansprüche, **dadurch gekennzeichnet, dass** das Flockmaterial als Pulver oder Fasern, insbesondere als zerkleinerte oder gemahlene Fasern, ausgebildet ist.

5. Medizinprodukt nach einem der vorhergehenden Ansprüche, **dadurch gekennzeichnet, dass** das Flockmaterial in einer musterartigen Anordnung auf dem mindestens einen Produktkörper, insbesondere auf der Klebstoffschicht, vorgesehen ist, wobei die Anordnung bevorzugt ausgewählt ist aus der Gruppe bestehend aus einer linearen Anordnung, versetzten Anordnung, schneckenförmigen oder spiralförmigen Anordnung, mäanderförmigen Anordnung, serpentinenartigen Anordnung, statistischer oder unregelmäßiger Anordnung, ringförmiger Anordnung und Kombinationen davon.

6. Medizinprodukt nach einem der vorhergehenden Ansprüche, **dadurch gekennzeichnet, dass** das Flockmaterial ein Material beinhaltet oder daraus gebildet ist, das ausgewählt ist aus der Gruppe bestehend aus Polyolefinen, Polyvinylalkoholen, Polyamiden, Polyimiden, Polyestern, Polyurethanen, insbesondere thermoplastischen Polyurethanen, Polyhydroxyalkanoaten, Proteinen, Polysacchariden, Copolymeren davon, Stereoisomeren davon, Salzen davon und Kombinationen davon, wobei das Flockmaterial besonders bevorzugt ein Material beinhaltet oder daraus gebildet ist, das ausgewählt ist aus der Gruppe bestehend aus Polyethylen, Polyethylen niedriger Dichte, Polyethylen hoher Dichte, hochmolekularem Polyethylen, ultrahochmolekularem Polyethylen, Polypropylen, Polyethylenterephthalat, Polypropylenterephthalat, Polybutylenterephthalat, Polyamid 6, Polyamid 6-6, Polyamid 6-12, Polyamid 12, Rayon, Seide, insbesondere Spinnenseide, Polytetrafluorethylen, Polyvinylidendichlorid, Polyvinylidendifluorid, Polytetrafluorpropylen, Polyhexafluorpropylen, Polyvinylalkohol, Polyglycolid, Polylactid, Polydioxanon, Polyhydroxybutyrat, Poly-3-hydroxybutyrat, Poly-4-hydroxybutyrat, Polytrimethylencarbonat, Poly-ε-caprolacton, Proteinen wie Kollagen, Gelatine, Elastin, Reticulin, Fibronectin, Laminin, Fibrin, Fibrinogen, Albumin, Protein wie Stärke, Amylose, Amylopectin, Dextran, Cellulose, Methylcellulose, Carboxymethylcellulose, Chitosan, Hyaluronsäure, Dextransulfat, Heparin, Heparansulfat, Chondroitinsulfat, Dermatansulfat, Copolymeren davon, Stereoisomeren davon, Salzen davon und Kombinationen davon.

7. Medizinprodukt nach einem der vorhergehenden Ansprüche, **dadurch gekennzeichnet, dass** das Flockmaterial einen Wirkstoff, insbesondere einen kosmetischen, pharmazeutischen und/oder medizinischen Wirkstoff, beinhaltet oder daraus gebildet ist, der bevorzugt ausgewählt ist aus der Gruppe bestehend aus Antibiotika, Desinfektionsmitteln, onkologischen Wirkstoffen, Antinarbenmitteln wie Captopril, Entzündungshemmern, Schmerzmitteln, Wachstumsfaktoren, Zelldifferenzierungsfaktoren, Zelladhäsionsfaktoren, Zellrekrutierungsfaktoren, Zellrezeptoren, Zellbindungssequenzen, Cytokinen, Peptiden, Proteinen wie Kollagen, Lipiden, Polysacchariden wie Hyaluronsäuren, Oligonukleotiden, Polynukleotiden, DNA, RNA, Stereosisomeren davon, Salzen davon und Kombinationen davon.

8. Medizinprodukt nach einem der vorhergehenden Ansprüche, **dadurch gekennzeichnet, dass** der mindestens eine Produktkörper ein Material beinhaltet oder daraus gebildet ist, das ausgewählt ist aus der Gruppe bestehend aus Polyolefinen, Polyamiden, Polyestern, Polyurethanen, Polyhydroxyalkanoaten, Proteinen, Polysacchariden, Copolymeren davon, Stereoisomeren davon, Salzen davon und Kombinationen davon, wobei der mindestens eine Produktkörper besonders bevorzugt ein Material beinhaltet oder daraus gebildet ist, das ausgewählt ist aus der Gruppe bestehend aus Polyethylen, Polyethylen niedriger Dichte, Polyethylen hoher Dichte, hochmolekularem Polyethylen, ultrahochmolekularem Polyethylen, Polypropylen, Polyethylenterephthalat, Polypropylenterephthalat, Polybutylenterephthalat, Polyamid 6, Polyamid 6-6, Polyamid 6-12, Polyamid 12, Rayon, Seide, insbesondere Spinnenseide, Polytetrafluorethylen, Polyvinylidendichlorid, Polyvinylidendifluorid, Polytetrafluorpropylen, Polyhexafluorpropylen, Polyvinylalkohol, Polyglycolid, Polylactid, Polydioxanon, Polyhydroxybutyrat, Poly-3-hydroxybutyrat, Poly-4-hydroxybutyrat, Polytrimethylencarbonat, Poly-ε-caprolacton, Kollagen, Gelatine, Elastin, Fibronectin, Laminin, Fibrin, Albumin, Stärke, Amylose, Amylopectin, Dextran, Cellulose, Methylcellulose, Carboxymethylcellulose, Chitosan, Hyaluronsäure, Dextransulfat, Heparin, Heparansulfat, Chondroitinsulfat, Dermatansulfat, Copolymeren davon, Stereoisomeren davon, Salzen davon und Kombinationen davon.

9. Medizinprodukt nach einem der vorhergehenden Ansprüche, **dadurch gekennzeichnet, dass** der mindestens eine Produktkörper als textiles Flächengebilde ausgebildet ist, das bevorzugt ausgewählt ist aus der Gruppe bestehend aus Gewebe, Maschenware, insbesondere Kettengewirk, Vlies (Nonwoven), insbesondere Sprühvlies, Filz, Verbundstoffen davon und Kombinationen davon.

10. Medizinprodukt nach einem der vorhergehenden Ansprüche, **dadurch gekennzeichnet, dass** der mindestens eine Produktkörper als Netz oder als offenmaschige Struktur, insbesondere als offenmaschiges textiles Flächengebilde, bevorzugt als offenmaschiges Gewirk, ausgebildet ist.

11. Medizinprodukt nach einem der Ansprüche 1 bis 8, **dadurch gekennzeichnet, dass** der mindestens eine Produktkörper als nicht-textiler Körper, bevorzugt ausgewählt aus der Gruppe bestehend aus Membran, Bahn, Patch, Pad, Schaum, Schwamm, Folie, Film, Beschichtung und Kombinationen, insbesondere Verbundstoffen daraus, ausgebildet ist.

12. Medizinprodukt nach einem der vorhergehenden Ansprüche, **dadurch gekennzeichnet, dass** das Produkt als chirurgisches Implantat ausgebildet ist.

13. Medizinprodukt nach einem der vorhergehenden Ansprüche, **dadurch gekennzeichnet, dass** das Medizinprodukt ausgewählt ist aus der Gruppe bestehend aus Hernienimplantat, insbesondere Herniennetz, Prolapsimplantat, insbesondere Prolapsnetz, Inkontinenzimplantat, insbesondere Inkontinenznetz, Shunt, Katheter, Gewebegerüst, Stent, Stentgraft, Gefäßgraft, insbesondere Arteriengraft, Aortengraft, Hüftprothese, Knieprothese, Knorpelersatz, Faszienersatz, Zellträger, Dura-Mater-Onlay, Anastomosenprodukt, Wundauflage und Hämostase-Patch.

14. Verfahren zur Herstellung eines Medizinprodukts nach einem der vorhergehenden Ansprüche, umfassend die Schritte
a) Beschichten mindestens eines Produktkörpers mit einem Klebstoff,
b) Beflocken des mindestens einen mit Klebstoff beschichteten Produktkörpers mit einem Flockmaterial, während der Klebstoff noch aktiv, insbesondere nass, ist oder nach Aktivierung des Klebstoffs.

15. Verfahren zur Herstellung eines Medizinprodukts nach einem der Ansprüche 1 bis 13, umfassend die Schritte
a) Bereitstellen mindestens eines Produktkörpers, der mindestens teilweise aus einem Material gebildet ist, das nach Aktivierung als Klebstoff wirkt,
b) Beflocken des mindestens einen Produktkörpers mit einem Flockmaterial nach Aktivierung des Materials des mindestens einen Produktkörpers.

16. Chirurgische Kombination, insbesondere chirurgisches Kit, umfassend ein Medizinprodukt nach einem der Ansprüche 1 bis 13 und mindestens ein Einführinstrument.

## Revendications

1. Dispositif médical comprenant au moins un corps de dispositif et un matériau de flocage, **caractérisé en ce que** le dispositif médical comprend en outre une couche d'adhésif, le matériau de flocage étant fixé à la couche d'adhésif.

2. ispositif médical selon la revendication 1, **caractérisé en ce que** la couche d'adhésif se compose d'un matériau, en particulier un polymère, ayant une température de fusion inférieure à celle d'un matériau, en particulier un polymère, dont se compose ledit au moins un corps de dispositif.

3. Dispositif médical selon la revendication 1 ou 2, **caractérisé en ce que** la couche d'adhésif contient ou se compose d'un matériau sélectionné dans le groupe constitué des matériaux thermofusibles, de l'alcool polyvinylique, de l'acétate de polyvinyle, du chlorure de polyvinyle, du propionate de polyvinyle, des polyacrylates, des résines époxydes, des résines acryliques, des encollages de résine tels qu'un encollage de résine d'urée, des polyuréthanes, des cires, des polyhydroxyalcanoates, de protéines telles que le collagène, la gélatine, l'élastine, la fibronectine et des matériaux similaires, des polysaccharides, de copolymères de ceux-ci, de stéréoisomères de ceux-ci, de sels de ceux-ci, et de combinaisons de ceux-ci.

4. Dispositif médical selon l'une quelconque des revendications précédentes, **caractérisé en ce que** le matériau de flocage est sous forme de poudre ou de fibres, en particulier sous forme de fibres écrasées ou broyées.

5. Dispositif médical selon l'une quelconque des revendications précédentes, **caractérisé en ce que** le matériau de flocage est arrangé selon une disposition similaire à un motif sur ledit au moins un corps de dispositif, en particulier sur la couche d'adhésif, la disposition étant préférablement sélectionnée dans le groupe constitué d'une disposition linéaire, d'une disposition décalée, d'une disposition hélicoïdale ou en spirale, d'une disposition sinueuse, d'une disposition onduleuse, d'une disposition aléatoire ou irrégulière, d'une disposition annulaire, et de combinaisons de celles-ci.

6. Dispositif médical selon l'une quelconque des revendications précédentes, **caractérisé en ce que** le matériau de flocage contient ou se compose d'un matériau qui est sélectionné dans le groupe constitué des polyoléfines, des alcools polyvinyliques, des polyamides, des polyimides, des polyesters, des polyuréthanes, en particulier des polyuréthanes thermoplastiques, des polyhydroxyalcanoates, des protéines, des polysaccharides, de copolymères de ceux-ci, de stéréoisomères de ceux-ci, de sels de ceux-ci, et de combinaisons de ceux-ci, le matériau de flocage contenant ou se composant plus préférablement d'un matériau qui est sélectionné dans le groupe constitué du polyéthylène, du polyéthylène de faible densité, du polyéthylène de haute densité, du polyéthylène à haute masse moléculaire, du polyéthylène à ultra-haute masse moléculaire, du polypropylène, du téréphtalate de polyéthylène, du téréphtalate de polypropylène, du téréphtalate de polybutylène, du polyamide 6, du polyamide 6-6, du polyamide 6-12, du polyamide 12, de la rayonne, de la soie, en particulier la soie d'araignée, du polytétrafluoroéthylène, du dichlorure de polyvinylidène, du difluorure de polyvinylidène, du polytétrafluoropropylène, du polyhexafluoropropylène, de l'alcool polyvinylique, du polyglycolide, du polylactide, de la polydioxanone, du polyhydroxybutyrate, du poly-3-hydroxybutyrate, du poly-4-hydroxybutyrate, du carbonate de polytriméthylène, de la poly-ε-caprolactone, de protéines telles que le collagène, la gélatine, l'élastine, la réticuline, la fibronectine, la laminine, la fibrine, le fibrinogène, l'albumine, de protéine tels que l'amidon, l'amylose, l'amylopectine, le dextrane, la cellulose, la méthylcellulose, la carboxyméthylcellulose, du chitosane, de l'acide hyaluronique, du sulfate de dextrane, de l'héparine, du sulfate d'héparane, du sulfate de chondroïtine, du sulfate de dermatane, de copolymères de ceux-ci, de stéréoisomères de ceux-ci, de sels de ceux-ci, et de combinaisons de ceux-ci.

7. Dispositif médical selon l'une quelconque des revendications précédentes, **caractérisé en ce que** le matériau de flocage contient ou se compose d'un agent actif, en particulier un agent cosmétique, pharmaceutique et/ou médicinal, préférablement sélectionné dans le groupe constitué des antibiotiques, des désinfectants, des agents oncologiques, des agents inhibant la formation de cicatrices tels que le captopril, des anti-inflammatoires, des analgésiques, des facteurs de croissance, des facteurs de différenciation cellulaire, des facteurs d'adhésion cellulaire, des facteurs de recrutement cellulaire, des récepteurs cellulaires, des séquences de liaisons cellulaires, des cytokines, des peptides, de protéines telles qu'un collagène, des lipides, de polysaccharides tels que les acides hyaluroniques, des oligonucléotides, des polynucléotides, de l'ADN, de l'ARN, de stéréoisomères de ceux-ci, de sels de ceux-ci, et de combinaisons de ceux-ci.

8. Dispositif médical selon l'une quelconque des revendications précédentes, **caractérisé en ce que** ledit au moins un corps de dispositif contient ou se compose d'un matériau sélectionné dans le groupe constitué des polyoléfines, des polyamides, des polyesters, des polyuréthanes, des polyhydroxyalcanoates, des protéines, des polysaccharides, de copolymères de ceux-ci, de stéréoisomères de ceux-ci, de sels de ceux-ci, et de combinaisons de ceux-ci, ledit au moins un corps de dispositif contenant ou se composant plus préférablement d'un matériau qui est sélectionné dans le groupe constitué du polyéthylène, du polyéthylène de faible densité, du polyéthylène de haute densité, du polyéthylène à haute masse moléculaire, du polyéthylène à ultra-haute masse moléculaire, du polypropylène, du téréphtalate de polyéthylène, du téréphtalate de polypropylène, du téréphtalate de polybutylène, du polyamide 6, du polyamide 6-6, du polyamide 6-12, du polyamide 12, de la rayonne, de la soie, en particulier la soie d'araignée, du polytétrafluoroéthylène, du dichlorure de polyvinylidène, du difluorure de polyvinylidène, du polytétrafluoropropylène, du polyhexafluoropropylène, de l'alcool polyvinylique, du polyglycolide, du polylactide, de la polydioxanone, du polyhydroxybutyrate, du poly-3-hydroxybutyrate, du poly-4-hydroxybutyrate, du carbonate de polytriméthylène, de la poly-ε-caprolactone, du collagène, de la gélatine, de l'élastine, de la fibronectine, de la laminine, de la fibrine, de l'albumine, de l'amidon, de l'amylose, de l'amylopectine, du dextrane, de la cellulose, de la méthylcellulose, de la carboxyméthylcellulose, du chitosane, de l'acide hyaluronique, du sulfate de dextrane, de l'héparine, du sulfate d'héparane, du sulfate de chondroïtine, du sulfate de dermatane, de copolymères de ceux-ci, de stéréoisomères de ceux-ci, de sels de ceux-ci, et de combinaisons de ceux-ci.

9. Dispositif médical selon l'une quelconque des revendications précédentes, **caractérisé en ce que** ledit au moins un corps de dispositif est sous la forme d'un textile qui est préférablement sélectionné dans le groupe constitué d'un tissu tissé, d'un tricot, en particulier un tricot à mailles jetées, d'un tissu non tissé, en particulier un tissu non tissé ayant reçu une pulvérisation, d'un feutre, de composites de ceux-ci, et de combinaisons de ceux-ci.

10. Dispositif médical selon l'une quelconque des revendications précédentes, **caractérisé en ce que** ledit au moins un corps de dispositif est un filet ou un tissu à mailles ouvertes, en particulier un textile à mailles ouvertes, préférablement un tricot à mailles ouvertes.

11. Dispositif médical selon l'une quelconque des revendications 1 à 8, **caractérisé en ce que** ledit au moins un corps de dispositif est sous la forme d'un corps non textile, préférablement sélectionné dans le groupe constitué d'une membrane, d'une feuille, d'un patch, d'une plaquette, d'une mousse, d'une éponge, d'une feuille métallique, d'un film, d'un revêtement, et de combinaisons, en particulier de composites, de ceux-ci.

12. Dispositif médical selon l'une quelconque des revendications précédentes, **caractérisé en ce que** le dispositif est un implant chirurgical.

13. Dispositif médical selon l'une quelconque des revendications précédentes, **caractérisé en ce que** le dispositif médical est sélectionné dans le groupe constitué d'un implant herniaire, en particulier un filet herniaire, d'un implant destiné au traitement d'un prolapsus, en particulier un filet destiné au traitement d'un prolapsus, d'un implant destiné au traitement d'une incontinence, en particulier un filet destiné au traitement d'une incontinence, d'un shunt, d'un cathéter, d'un support tissulaire, d'un stent, d'un stent-greffe, d'une greffe vasculaire, en particulier une greffe artérielle, une greffe aortique, d'une prothèse de hanche, d'une prothèse de genou, d'un substitut de cartilage, d'un substitut de fascia, d'un support cellulaire, d'un implant de greffe durale, d'un dispositif anastomotique, d'un pansement de plaie et d'un patch hémostatique.

14. Procédé de fabrication d'un dispositif médical selon l'une quelconque des revendications précédentes, comprenant les étapes qui consistent à :
a) revêtir d'une couche d'adhésif au moins un corps de dispositif,
b) floquer ledit au moins un corps de dispositif revêtu d'une couche d'adhésif avec un matériau de flocage alors que l'adhésif est encore actif, en particulier mouillé, ou au moment de l'activation de l'adhésif.

15. Procédé de fabrication d'un dispositif médical selon l'une quelconque des revendications 1 à 13, comprenant les étapes qui consistent à :
a) fournir au moins un corps de dispositif qui est constitué au moins en partie d'un matériau qui agit comme un adhésif quand il est activé,
b) floquer ledit au moins un corps de dispositif avec un matériau de flocage au moment de l'activation du matériau dudit au moins un corps de dispositif.

16. Combinaison chirurgicale, en particulier trousse chirurgicale, comprenant un dispositif médical selon l'une quelconque des revendications 1 à 13, et au moins un instrument de mise en place.
